(19) Europäisches Patentamt
European Patent Office
Office européen des brevets

(11) Veröffentlichungsnummer: **0 291 798 B1**

# (12) EUROPÄISCHE PATENTSCHRIFT

(45) Veröffentlichungstag der Patentschrift: **18.03.92**

(21) Anmeldenummer: **88107381.1**

(22) Anmeldetag: **07.05.88**

Die Akte enthält technische Angaben, die nach dem Eingang der Anmeldung eingereicht wurden und die nicht in dieser Patentschrift enthalten sind.

(51) Int. Cl.5: **C07D 249/08**, A01N 43/653, C07D 303/22, C07C 43/20, C07C 321/24, C07D 303/34

## (54) Hydroxyalkyl-triazolyl-Derivate.

(30) Priorität: **18.05.87 DE 3716560**

(43) Veröffentlichungstag der Anmeldung:
**23.11.88 Patentblatt 88/47**

(45) Bekanntmachung des Hinweises auf die Patenterteilung:
**18.03.92 Patentblatt 92/12**

(84) Benannte Vertragsstaaten:
**BE CH DE FR GB IT LI NL**

(56) Entgegenhaltungen:
**EP-A- 0 040 345**
**EP-A- 0 061 835**
**EP-A- 0 091 398**
**EP-A- 0 181 529**

(73) Patentinhaber: **BAYER AG**

**W-5090 Leverkusen 1 Bayerwerk(DE)**

(72) Erfinder: **Kraatz, Udo, Dr.**
**Andreasstrasse 22a**
**W-5090 Leverkusen(DE)**
Erfinder: **Holmwood, Graham, Dr.**
**Krutscheider Weg 105**
**W-5600 Wuppertal 11(DE)**
Erfinder: **Büchel, Karl Heinz, Prof. Dr.**
**Dabringhausener Strasse 42**
**W-5093 Burscheid(DE)**
Erfinder: **Brandes, Wilhelm, Dr.**
**Eichendorffstrasse 3**
**W-5653 Leichlingen(DE)**
Erfinder: **Dutzmann, Stefan, Dr.**
**Leinenweberweg 33**
**W-4000 Düsseldorf 13(DE)**

EP 0 291 798 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Hydroxyalkyltriazolyl-Derivate, ein Verfahren zu ihrer Herstellung sowie ihre Verwendung als Fungizide und Pflanzenwachstumsregulatoren.

Es ist bereits bekannt, daß zahlreiche Hydroxyalkylazolyl-Derivate fungizide und pflanzenwachstumsregulierende Eigenschaften besitzen (vgl. EP-A 0 040 345 und EP-A 0 061 835). So läßt sich z.B. 2-(4-Chlorphenoxy-methyl)-3,3-dimethyl-1-(1,2,4-triazol-l-yl)-butan-2-ol zur Bekämpfung von Pilzen und zur Regulierung des Pflanzenwachstums einsetzen. Die Wirksamkeit dieses Stoffes ist sehr gut; allerdings läßt die Pflanzenverträglichkeit in manchen Fällen zu wünschen übrig.

Aus der EP-A 0 091 398 sind ebenfalls bestimmte Hydroxyalkyl-azolyl-Derivate mit fungiziden und pflanzenwuchsregulierenden Eigenschaften bekannt. Es werden jedoch keine Verbindungen offenbart, in denen der Azolyl-Rest über eine $CH_2$-Gruppe mit dem verbleibenden Molekülteil verbunden ist.

Ferner werden auch in der EP-A 0 181 529 Azolylcarbinol-Derivate beschrieben, in denen ein gegebenenfalls substituierter Phenyl-Rest über eine $-OCH_2$-oder $-SCH_2$-Brücke mit dem Carbinol-Kohlenstoffatom verknüpft ist. In keiner der Verbindungen ist aber eine $(CH_3)_2CH-CH_2-$ oder $CH_3-(CH_2)_4-$ Gruppierung oder ein Alkylrest mit mehr als 5 Kohlenstoffatomen als Substituent am zentralen Kohlenstoffatom enthalten.

Es wurden nun neue Hydroxyalkyl-triazolyl-Derivate der Formel

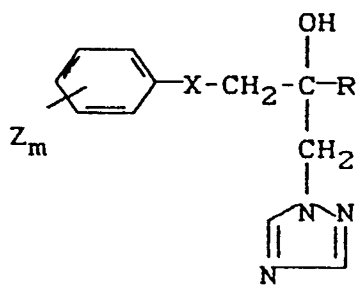

(I)

in welcher

R für die Reste der Formeln $-CH_2-CH(CH_3)_2$, $-(CH_2)_4-CH_3$,

$-(CH_2)_5-CH_3$, $-(CH_2)_6-CH_3$, 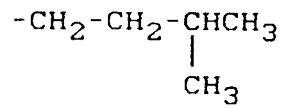,

$-(CH_2)_8CH_3$

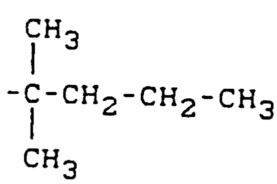 oder 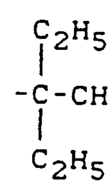 steht,

X für Sauerstoff oder Schwefel steht,

Z für Fluor, Chlor, Brom, Methyl, Ethyl, Trifluormethyl, Trifluormethoxy, Trifluormethylthio, Phenyl, Methoximinomethyl oder Ethoximinomethyl steht und

m für die Zahlen 0, 1, 2 oder 3 steht,

sowie deren Säureadditions-Salze und Metallsalz-Komplexe gefunden.

Die neuen Hydroxyalkyl-triazolyl-Derivate der Formel (I) besitzen mindestens ein asymmetrisch substituiertes Kohlenstoffatom und können deshalb in optischen Isomerenformen anfallen. Die Erfindung betrifft

sowohl die Racemate als auch die einzelnen Isomeren und deren Gemische.

Weiterhin wurde gefunden, daß man Hydroxyalkyl-triazolyl-Derivate der Formel (I) sowie deren Säureadditions-Salze und Metallsalz-Komplexe erhält, wenn man Oxirane der Formel

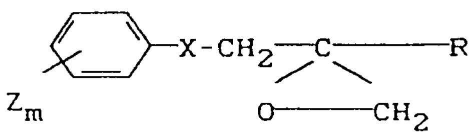

(II)

in welcher

R, X, Z und m die oben angegebene Bedeutung haben,

mit 1,2,4-Triazol der Formel

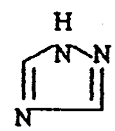

(III)

in Gegenwart eins Verdünnungsmittels und gegebenenfalls in Gegenwart eines Säurebindemittels sowie gegebenenfalls in Gegenwart eines Katlysators umsetzt und gegebenenfalls anschließend an die so erhaltenen Verbindungen der Formel (I) eine Säure oder ein Metallsalz addiert.

Schließlich wurde gefunden, daß sich die Hydroxyalkyltriazolyl-Derivate der Formel (I) sowie deren Säureadditions-Salze und Metallsalz-Komplexe durch sehr gute fungizide und pflanzenwachstumsregulierende Eigenschaften auszeichnen.

Überraschenderweise besitzen die erfindungsgemäßen Stoffe eine bessere fungizide und pflanzenwachstumsregulierende Wirksamkeit als 1-(4-Chlorphenoxy-methyl)-3,3-dimethyl-1-(1,2,4-triazol-1-yl)-butan-2-ol welches ein konstitutionell ähnlicher, vorbekannter Wirkstoff gleicher Wirkungsrichtung ist. Außerdem weisen die erfindungsgemäßen Stoffe eine hervorragende Pflanzenverträglichkeit auf.

Die erfindungsgemäßen Hydroxyalkyl-triazolyl-Derivate sind durch die Formel (I) allgemein definiert.

Wenn m für 2 oder 3 steht, können die Substituenten für Z gleich oder verschieden sein.

Bevorzugte Säureadditions-Salze sind Additionsprodukte von Hydroxyalkyl-triazolyl-Derivaten der Formel (I) mit Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionellen Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Bevorzugte Metallsalz-komplexe sind Additionsprodukte aus Salzen von Metallen der II. bis IV. Haupt- und der I. und II. sowie IV. bis VIII. Nebengruppe des Periodensystems der Elemente und denjenigen Hydroxyalkyl-triazoyl-Derivaten der Formel (I), in denen R, X, Z und m die oben angegebenen Bedeutungen haben Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenphysiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Verbindungen seien die in der folgenden Tabelle aufgeführten Stoffe genannt.

**Tabelle:**

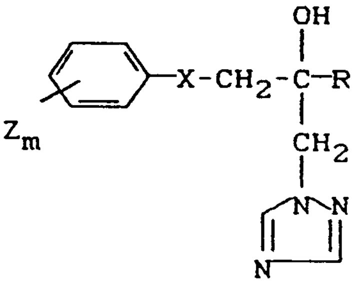

(I)

| $Z_m$ | R | X |
|---|---|---|
| $4-CF_3O-$ | $-(CH_2)_4-CH_3$ | O |
| $2,4-Cl_2$ | $-(CH_2)_4-CH_3$ | O |
| 4-F | $-(CH_2)_4-CH_3$ | O |
| $4-CH=N-OCH_3$ | $-(CH_2)_4-CH_3$ | O |
| $2,6-Cl_2$ | $-(CH_2)_4-CH_3$ | O |
| 4— 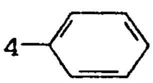 | $-(CH_2)_4-CH_3$ | O |
| $4-CF_3$ | $-(CH_2)_4-CH_3$ | O |
| $4-CH_3$ | $-(CH_2)_4-CH_3$ | O |
| 4-Cl | $-(CH_2)_4-CH_3$ | S |
| $4-CH=N-OCH_3$ | $-(CH_2)_4-CH_3$ | S |
| $2,4-Cl_2$ | $-(CH_2)_5-CH_3$ | O |
| 4-F | $-(CH_2)_5-CH_3$ | O |
| $4-CH=N-OCH_3$ | $-(CH_2)_5-CH_3$ | O |
| $2,6-Cl_2$ | $-(CH_2)_5-CH_3$ | O |
| 4— 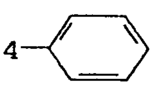 | $-(CH_2)_5-CH_3$ | O |
| 4-Cl | $-(CH_2)_5-CH_3$ | O |
| $4-CF_3$ | $-(CH_2)_5-CH_3$ | O |
| $4-CH_3$ | $-(CH_2)_5-CH_3$ | O |
| $2,4-Cl_2$ | $-(CH_2)_6-CH_3$ | O |
| 4-F | $-(CH_2)_6-CH_3$ | O |
| $4-CH=N-OCH_3$ | $-(CH_2)_6-CH_3$ | O |
| $2,6-Cl_2$ | $-(CH_2)_6-CH_3$ | O |

**Tabelle:** (Fortsetzung)

| $Z_m$ | R | X |
|---|---|---|
| 4 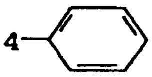 | $-(CH_2)_6-CH_3$ | O |
| - | $-(CH_2)_6-CH_3$ | O |
| $4-CF_3$ | $-(CH_2)_6-CH_3$ | O |
| $4-CH_3$ | $-(CH_2)_6-CH_3$ | O |
| $2,4-Cl_2$ | $CH_3$<br>$-CH_2-CH_2-CH-CH_3$ | O |
| 4-F | $CH_3$<br>$-CH_2-CH_2-CH-CH_3$ | O |
| $4-CH=N-OCH_3$ | $CH_3$<br>$-CH_2-CH_2-CH-CH_3$ | O |
| $2,6-Cl_2$ | $CH_3$<br>$-CH_2-CH_2-CH-CH_3$ | O |
| 4 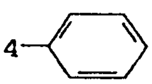 | $CH_3$<br>$-CH_2-CH_2-CH-CH_3$ | O |
| - | $CH_3$<br>$-CH_2-CH_2-CH$<br>$CH_3$ | O |
| $4-CF_3$ | $CH_3$<br>$-CH_2-CH_2-CH-CH_3$ | O |
| $4-CF_3O-$ | $CH_3$<br>$-CH_2-CH_2-CH$<br>$CH_3$ | O |
| $4-CH_3$ | $CH_3$<br>$-CH_2-CH_2-CH-CH_3$ | O |

**Tabelle:** (Fortsetzung)

| $Z_m$ | R | X |
|---|---|---|
| 2,4-$Cl_2$ | 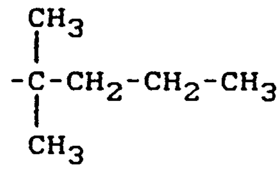 | O |
| 4-Cl | $-(CH_2)_8CH_3$ | O |
| 2,4-$Cl_2$ | $-(CH_2)_8CH_3$ | O |
| 4-F | 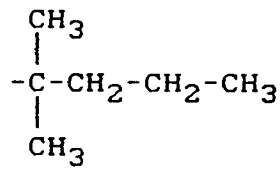 | O |
| 4-CH=N-$OCH_3$ | 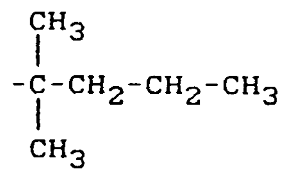 | O |
| 2,6-$Cl_2$ | 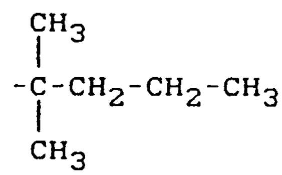 | O |
| 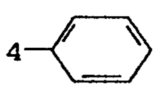 | 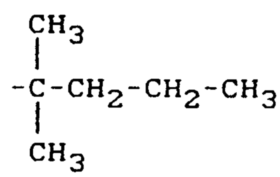 | O |
| - | 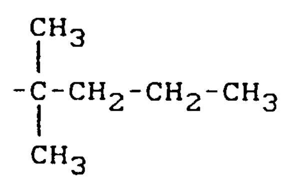 | O |
| 4-$CF_3$ | 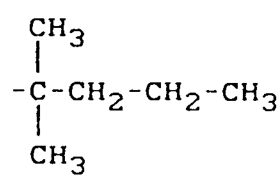 | O |

Tabelle: (Fortsetzung)

| $Z_m$ | R | X |
|---|---|---|
| $4-CH_3$ | 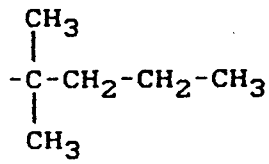 | O |
| 4-Cl | $-CH_2-CH(CH_3)_2$ | S |
| $2,4-Cl_2$ | $-CH_2-CH(CH_3)_2$ | O |
| $2,4-Cl_2$ | $-CH_2-CH(CH_3)_2$ | S |
| 4-F | $-CH_2-CH(CH_3)_2$ | O |
| 4-F | $-CH_2-CH(CH_3)_2$ | S |
| $4-CH=N-OCH_3$ | $-CH_2-CH(CH_3)_2$ | O |
| $4-CH=N-OCH_3$ | $-CH_2-CH(CH_3)_2$ | S |
| 4-Cl, $2-CH_3-$ | $-CH_2-CH(CH_3)_2$ | O |
| 4-Cl | 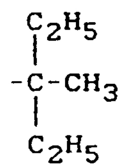 | O |
| 4-Cl | 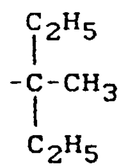 | S |
| 4-Cl | $-(CH_2)_5-CH_3$ | S |
| 4-Cl | $-(CH_2)_6-CH_3$ | S |
| 4-Cl | $-CH_2-CH_2-CH(CH_3)_2$ | S |
| 4-Cl | 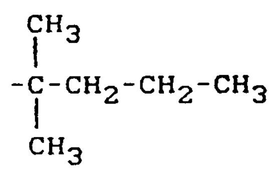 | S |
| $2,4-Cl_2$ | 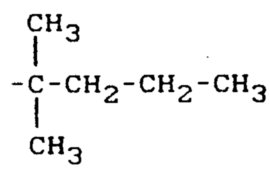 | S |
| $3-CF_3O$ | $-CH_2-CH(CH_3)_2$ | O |
| $4-CF_3O$ | $-CH_2-CH(CH_3)_2$ | O |

Verwendet man 2-(4-Chlor-phenoxy-methyl)-2-(2-methylprop-1-yl)-oxiran und 1,2,4-Triazol als Ausgangssubstanzen, so kann der Verlauf des erfindungsgemäßen Verfahrens durch das folgende Formelschema wiedergegeben werden:

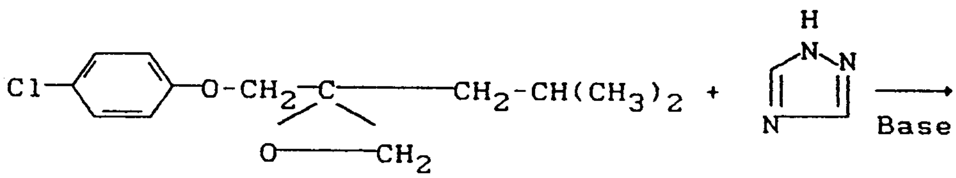

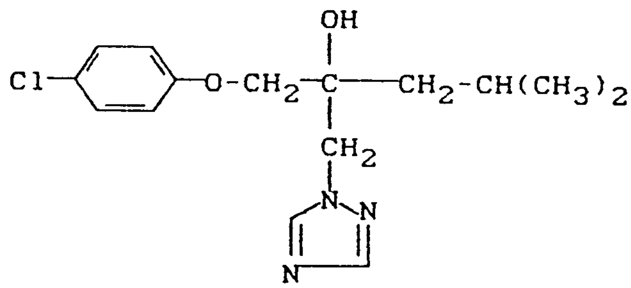

Die bei der Durchführung des erfindungsgemäßen Verfahrens als Ausgangsstoffe zu verwendenden Oxirane sind durch die Formel (II) allgemein definiert.

Die Oxirane der Formel (II) sind noch nicht bekannt. Sie lassen sich herstellen, indem man

a) in einer ersten Stufe Ketone der Formel

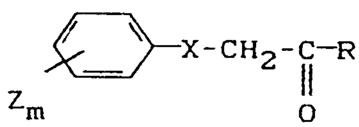

(IV)

in welcher
R, X, Z und m die oben angegebene Bedeutung haben,
mit Methyl-triphenyl-phosphoniumbromid der Formel

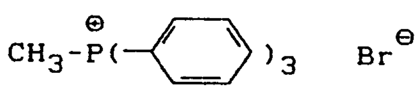

(V)

in Gegenwart einer Base und in Gegenwart eines Verdünnungsmittels umsetzt und dann
in einer zweiten Stufe die so erhaltenen Verbindungen der Formel

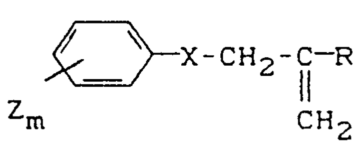

(VI)

in welcher
R, X, Z und m die oben angegebene Bedeutung haben,
mit Persäuren in Gegenwart eines Verdünnungsmittels umsetzt,

oder

b) Ketone der Formel

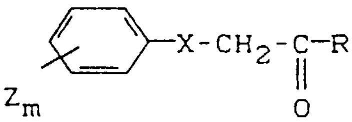

(IV)

in welcher
R, X, Z und m die oben angegebene Bedeutung haben,
entweder
α) mit Dimethyloxosulfonium-methylid der Formel

$$(CH_3)_2\overset{\delta\ominus}{S}O-\overset{\delta\ominus}{C}H_2 \qquad (VII)$$

oder
β) mit Dimethylsulfonium-methylid der Formel

$$(CH_3)_2\overset{\delta\ominus}{S}—\overset{\delta\ominus}{C}H_2 \qquad (VIII)$$

in Gegenwart eines Verdünnungsmittels umsetzt.

Die bei der Herstellung der Oxirane der Formel (II) als Ausgangsstoffe benötigten Ketone der Formel (IV) sind bekannt oder lassen sich nach im Prinzip bekannten Verfahren in einfacher Weise herstellen (vgl. EP-A 0 084 834). So erhält man Ketone der Formel (IV) dadurch, daß man Halogenketone der Formel

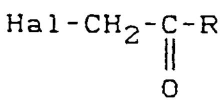

(IX)

in welcher
R die oben angegebene Bedeutung hat
und
Hal für Chlor oder Brom steht,
mit Phenolen oder Thiophenolen der Formel

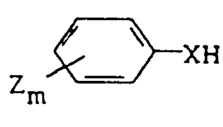

(X)

in welcher
X für Sauerstoff oder Schwefel steht und
Z und m die oben angegebene Bedeutung haben,
in Gegenwart einer Base, wie z.B. Kaliumcarbonat oder Triethylamin und in Gegenwart eines Verdünnungsmittels, wie z.B. Aceton oder Acetonitril, bei Temperaturen zwischen 20 °C und 100 °C umsetzt.

Das bei der Herstellung der Oxirane der Formel (II) nach dem Verfahren (a) weiterhin als Ausgangsmaterial benötigte Methyl-triphenyl-phosphonium-bromid der Formel (V) ist bekannt.

Die bei der Herstellung der Oxirane der Formel (II) nach dem obigen Verfahren (a) in der zweiten Stufe

als Ausgangssubstanzen benötigten Verbindungen der Formel (VI) sind bisher noch nicht bekannt.

Bei dem Verfahren (a) zur Herstellung der Oxirane der Formel (II) arbeitet man in der ersten Stufe in Gegenwart einer Base. Dabei kommen als Basen alle üblicherweise für Wittig-Reaktionen dieser Art verwendbaren Basen in Betracht. Vorzugsweise verwendbar ist Kalium-tert.-butylat.

Als Verdünnungsmittel kommen bei der Durchführung der ersten Stufe des obigen Verfahrens (a) zur Herstellung der Oxirane der Formel (II) alle für derartige Umsetzungen üblichen organischen Solventien infrage. Vorzugsweise verwendbar sind aromatische Kohlenwasserstoffe, wie Benzol, Toluol und Xylol.

Bei der Durchführung der zweiten Stufe des obigen Verfahrens (a) zur Herstellung der Oxirane der Formel (II) kommen als Reagenzien zur Epoxidierung alle üblichen Persäuren in Betracht. Vorzugsweise verwendbar sind meta-Chlorperbenzoesäure und Peressigsäure. Ferner ist es auch möglich, ein Gemisch aus Essigsäure und Wasserstoffperoxid einzusetzen.

Als Verdünnungsmittel kommen bei der Durchführung der zweiten Stufe des obigen Verfahrens (a) zur Herstellung der Oxirane der Formel (II) alle für derartige Epoxidierungen üblichen Solventien infrage. Vorzugsweise verwendbar sind Chloroform, Methylenchlorid und Essigsäure.

Die Reaktionstemperaturen können bei der Durchführung des Verfahrens (a) zur Herstellung der Oxirane der Formel (II) innerhalb eines bestimmten Bereiches variiert werden. Im allgemeinen arbeitet man in der ersten Stufe bei Temperaturen zwischen 50 °C und 140 °C, vorzugsweise zwischen 80 °C und 120 °C. In der zweiten Stufe arbeitet man im allgemeinen zwischen 10 °C und 60 °C, vorzugsweise zwischen 20 °C und 50 °C.

Bei der Durchführung des Verfahrens (a) zur Herstellung der Oxirane der Formel (II) geht man im allgemeinen so vor, daß man in der ersten Stufe auf 1 Mol an Keton der Formel (IV) zwischen 1 und 3 Mol Methyl-triphenyl-phosphonium-bromid der Formel (V) sowie zwischen 1 und 3 Mol Base einsetzt. In der zweiten Stufe setzt man auf 1 Mol an einer Verbindung der Formel (VI) jeweils zwischen 1 und 2 Mol an Persäure ein. Die Aufarbeitung erfolgt jeweils nach üblichen Methoden.

Das bei dem Verfahren (b) als Reaktionskomponente benötigte Dimethyloxosulfoniummethylid der Formel (VII) ist bekannt (vgl. J. Amer. Chem. Soc. 87, 1363-1364 (1965)). Es wird bei der obigen Umsetzung in frisch zubereitetem Zustand verarbeitet, indem man es in situ durch Umsetzung von Trimethyloxo-sulfonium-iodid mit Natriumhydrid oder Natriumamid, insbesondere mit Kalium-tert.-butylat oder Natriummethylat, in Gegenwart eines Verdünnungsmittels erzeugt.

Das bei dem Verfahren (b) außerdem als Reaktionskomponente in Betracht kommende Dimethylsulfonium-methylid der Formel (VIII) ist ebenfalls bekannt (vgl. Heterocycles 8, 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (b) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasertoffe, wie Benzol, Toluol oder Xylol, sowie stark polare Lösungsmittel, wie Dimethylsulfoxid.

Die Reaktionstemperaturen können bei dem Verfahren (b) in einem größeren Bereich variiert werden. Im allgemeinen arbeitet man bei Temperaturen zwischen 0 und 100 °C, vorzugsweise zwischen 10 und 60 °C.

Bei der Durchführung des Verfahrens (b) setzt man auf 1 Mol an Keton der Formel (IV) vorzugsweise 1 bis 3 Mol an Dimethyloxosulfonium-methylid der Formel (VII) bzw. an Dimethylsulfonium-methylid der Formel (VIII) ein. Die Isolierung der Oxirane erfolgt nach üblichen Methoden.

Die Oxirane der Formel (II) können bei dem erfindungsgemäßen Verfahren gegbenenfalls ohne Isolierung direkt weiter umgesetzt werden.

Als Verdünnungsmittel kommen für das erfindungsgemäße Verfahren unter den Reaktionsbedingungen inerte organische Lösungsmittel infrage. Vorzugsweise verwendbar sind Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für das erfindungsgemäße Verfahren alle üblicherweise verwendbaren anorganischen und organischen Basen in Betracht. Vorzugsweise verwendbar sind Alkalimetallcarbonate, wie z.B. Natrium- und Kaliumcarbonat; Alkalimetallhydroxide, wie z.B. Natriumhydroxid; Alkalimetallalkoholate, wie z.B. Natrium- und Kaliummethylat und -ethylat; Alkalimetallhydride, wie z.B. Natriumhydrid; sowie niedere tertiäre Alkylamine, Cycloalkylamine und Aralkylamine, wie insbesondere Triethylamin.

Als Katalysatoren kommen bei der Durchführung des erfindungsgemäßen Verfahrens alle üblicherweise

für derartige Umsetzungen einsetzbaren Reaktionsbeschleuniger in Frage. Vorzugsweise verwendbar ist α,α'-Azo-isobutyro-nitril.

Die Reaktionstemperaturen können bei der Durchführung des erfindungsgemäßen Verfahrens in einem größeren Bereich variiert werden. Im allgemeinen arbeitet man bei Temperaturen zwischen 0 und 200 °C, vorzugsweise zwischen 60 und 150 °C.

Bei der Durchführung des erfindungsgemäßen Verfahrens arbeitet man im allgemeinen unter Normaldruck. Es ist jedoch auch möglich, unter erhöhtem oder vermindertem Druck zu arbeiten.

Bei der Durchführung des erfindungsgemäßen Verfahrens setzt man auf 1 Mol Oxiran der Formel (II) vorzugsweise 1 bis 2 Mol an 1,2,4-Triazol und gegebenenfalls 1 bis 2 Mol an Säurebindemittel ein. Die Aufarbeitung und die Isolierung der Endprodukte erfolgt nach üblichen Methoden.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetst zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;

Cercospora-Arten, wie Cercospora canescens;
Alternaria-Arten, wie Alternaria brassicae;
Pseudocercosporella-Arten, wie Pseudocercosporella herpotrichoides.

Die erfindungsgemäßen Stoffe sind besonders gut geeignet zur Bekämpfung von Getreidekrankheiten, wie Erysiphe, Puccinia, Leptosphaeria, Cochloiobolus, Pyrenophora und

Pseudocercosporella, sowie zur Bekämpfung von Reiskrankheiten, wie Pyricularia. Außerdem zeigen die erfindungsgemäßen Stoffe auch eine gute fungizide in vitro-Wirkung.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung

besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Stoffe geht aus den folgenden Beispielen hervor.

Herstellungsbeispiele

Beispiel 1

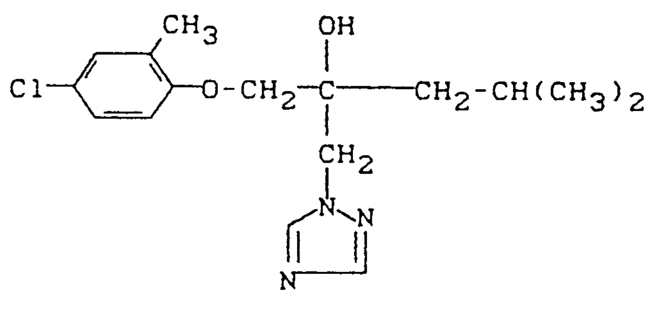

(I-1)

Eine Mischung aus 500,4 g (1,96 Mol) 1-(4-Chlor-2-methyl-phenoxy)-methyl-1-(2-methyl-propyl)-oxiran, 287 g (4,16 Mol) 1,2,4-Triazol, 296 g (2,14 Mol) Kaliumcarbonat und 1,5 1 Dimethylformamid wird 3 Stunden bei 70 ° C gerührt. Anschließend wird überschüssiges Lösungsmittel unter vermindertem Druck entfernt, der verbleibende ölige Rückstand mit Wasser und Methylenchlorid aufgenommen und die organische Phase abgetrennt. Man wäscht die organische Phase zweimal mit Wasser, trocknet dann und engt unter vermindertem Druck ein. Der Rückstand wird mit 500 ml n-Hexan und 150 ml Diisopropylether bei 20 ° C verrührt, wobei langsam Kristallisation eintritt. Der ausgeschiedene Feststoff wird abgesaugt und mit wenig n-Hexan/Diisopropylether (3:1) gewaschen. Man erhält auf diese Weise 242 g (38 % der Theorie) an 1-(4-Chlor-2-methylphenoxy)-2-hydroxy-2-(1,2,4-triazol-1-yl-methyl)-4-methyl-pentan in Form einer Festsubstanz vom Schmelzpunkt 118 ° C.

Herstellung von Ausgangsprodukten

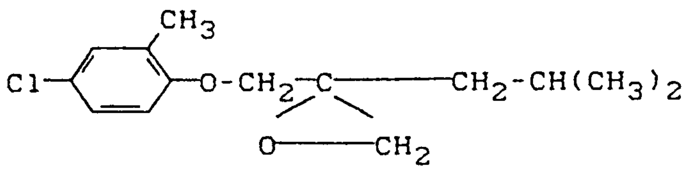

(II-1)

Zu einer Lösung von 538 g (2,5 Mol) Trimethyloxosulfonium-iodid in 1,7 1 Dimethylsulfoxid fügt man 280 g (2,5 Mol) Kalium-tert.-butylat und rührt 20 Min bei Raumtemperatur nach. Anschließend werden unter leichter Kühlung und unter Rühren 485,8 g (3,02 Mol) 1-(4-Chlor-2-methyl-phenoxy)-4-methyl-pentan-2-on hinzugetropft. Man rührt 4 Stunden bei 50 bis 60 °C, zieht dann die Hauptmenge an Lösungsmittel unter vermindertem Druck ab und verdünnt mit 2 Litern Wasser. Das Reaktionsgemisch wird mehrfach mit Methylenchlorid extrahiert. Die vereinigten organischen Phasen werden gewaschen und nach dem Trocknen unter vermindertem Druck eingeengt. Man erhält auf diese Weise 500,4 g (97,1 % der Theorie) an 1-(4-Chlor-2-methyl-phenoxy)-methyl-1-(2-methyl-propyl)-oxiran in Form eines viskosen Öles, das ohne zusätzliche Reinigung für die weitere Synthese verwendet wird.

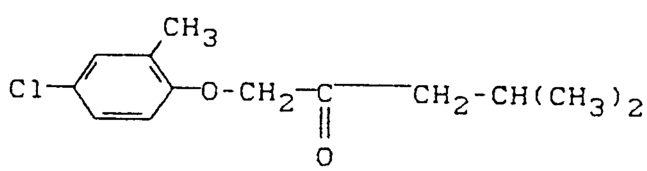

(IV-1)

Zu einer Suspension aus 2,9 Litern Aceton, 400 g Kaliumcarbonat und 417 g (2,9 Mol) 4-Chlor-2-methyl-phenol werden unter Rühren bei Raumtemperatur 410 g (2,3 Mol) Brommethyl-isobutylketon portionsweise hinzugefügt. Die Reaktion verläuft schwach exotherm, eine Kühlung ist aber nicht erforderlich. Nach beendeter Zugabe wird noch 3 Stunden unter Rückfluß erhitzt, dann abgesaugt und der Rückstand mit Aceton gewaschen. Die organische Phase wird unter vermindertem Druck eingeengt und der verbleibende Rückstand im Methylenchlorid aufgenommen. Die so erhaltene organische Phase wird zweimal mit 5 %iger wäßriger Natronlauge gewaschen, dann getrocknet und unter vermindertem Druck eingeengt. Man erhält auf diese Weise 530 g (96 % der Theorie) an 1-(4-Chlor-2-methyl-phenoxy)-4-methyl-pentan-2-on in Form eines hellgelben Öles, das ohne zusätzliche Reinigung für die weitere Synthese verwendet wird.

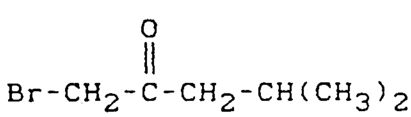

(IX-1)

Eine Lösung von 120,4 g (1,2 Mol) Isobutyl-methyl-keton in 800 ml Methanol wird bei 0 bis 5 °C unter Rühren innerhalb von 15 Minuten mit 224 g (71,3 ml = 1,4 Mol) Brom so versetzt, daß die Temperatur des Reaktionsgemisches 15 °C nicht übersteigt. Nach vollständiger Entfärbung fügt man 400 ml Wasser hinzu und rührt 16 Stunden bei Raumtemperatur. Danach wird das Reaktionsgemisch mit zusätzlichem Wasser verdünnt und dann mit Methylenchlorid extrahiert. Die organische Phase wird nacheinander mit Wasser und wäßriger Natriumhydrogencarbonat-Lösung gewaschen und unter vermindertem Druck eingeengt. Der Rückstand wird unter vermindertem Druck destilliert. Man erhält auf diese Weise 205 g (95 % der Theorie) an Brommethyl-isobutylketon in Form einer Flüssigkeit Kp. = 78-80 °C / 16 mbar.

In entsprechender Weise und gemäß den angegebenen Verfahrensbedingungen werden auch die in der folgenden Tabelle formelmäßig aufgeführten Verbindungen der Formel (I) hergestellt.

Tabelle

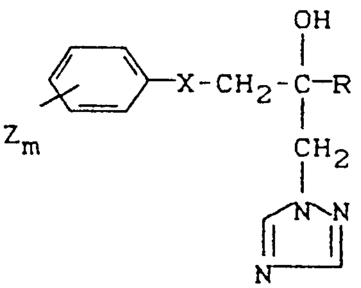

(I)

| Bsp. Nr. | Zm | X | R | Physikalische Konstante |
|---|---|---|---|---|
| 2 | 4-Cl | O | $-CH_2-CH(CH_3)_2$ | Harz |
| 3 | 4-Cl | S | $-CH_2-CH(CH_3)_2$ | " |
| 4 | 4-F | O | $-CH_2-CH(CH_3)_2$ | " |
| 5 | 2,4-$Cl_2$ | O | $-CH_2-CH(CH_3)_2$ | Fp. 85° C |
| 6 | 4-$CF_3$ | O | $-CH_2-CH(CH_3)_2$ | Fp. 99° C |
| 7 | 4-$CH=NOCH_3$ | O | $-CH_2-CH(CH_3)_2$ | Oil |
| 8 | 4-Cl | O | $-(CH_2)_4-CH_3$ | " |
| 9 | 4-Cl | O | $-(CH_2)_5-CH_3$ | Harz |
| 10 | 4-Cl | O | $-CH_2CH_2-CH(CH_3)_2$ | " |
| 11 | 4-F | O | $-CH_2CH_2-CH(CH_3)_2$ | $n_D^{24}$ : 1,5120 |
| 12 | 4-$CH=NOCH_3$ | O | $-CH_2CH_2-CH(CH_3)_2$ | $n_D^{24}$ : 1,5450 |

In den folgenden Verwendungsbeispielen wird die nachstehend angegebene Verbindung als Vergleichssubstanz eingesetzt.

(A) = 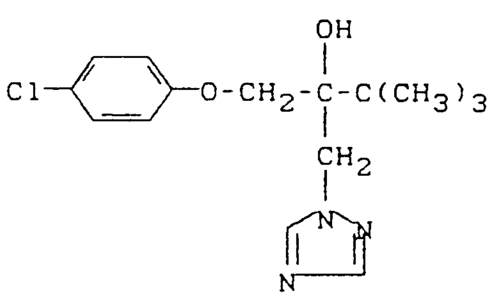

(bekannt aus EP-A 0 040 345)

Beispiel A

Pflanzenverträglichkeitstest

Testpflanze: Gurke
Versuchsdauer: 7 Tage
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Mit dieser Wirkstoffzubereitung bespritzt man junge Pflanzen bis zur Tropfnässe und stellt sie in einem Gewächshaus bei ca. 20 °C auf.

Die Pflanzen werden auf Schäden wie Wuchsbeeinträchtigungen, Verfärbungen und Nekrosen ausgewertet. Angegeben wird der Schädigungsgrad der Pflanzen in %. Dabei bedeutet:

0 %: keine Schäden
100 %: Pflanze vollkommen geschädigt.

In diesem Test zeigt die erfindungsgemäße Verbindung (I-1) eine bessere Verträglichkeit als die Vergleichssubstanz (A).

Beispiel B

Venturia-Test (Apfel) / kurativ

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspension des Apfelschorferregers (Venturia inaequalis) inokuliert. Die Pflanzen verbleiben 1 Tag bei 20 °C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigt die erfindungsgemäße Verbindung (I-1) eine bessere Wirkung als die Vergleichssubstanz (A).

**Patentansprüche**

**1.** Hydroxyalkyl-triazolyl-Derivate der Formel

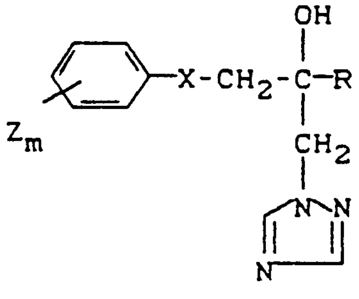

(I)

in welcher

R für die Reste der Formeln $-CH_2-CH(CH_3)_2$, $-(CH_2)_4-CH_3$,

$-(CH_2)_5-CH_3$, $-(CH_2)_6-CH_3$, 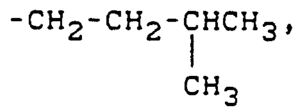,

$-(CH_2)_8CH_3$

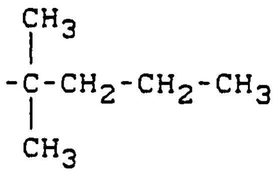 oder 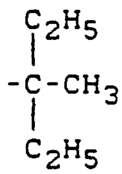 steht,

X für Sauerstoff oder Schwefel steht,

Z für Fluor, Chlor, Brom, Methyl, Ethyl, Trifluormethyl, Trifluormethoxy, Trifluormethylthio, Phenyl, Methoximinomethyl oder Ethoximinomethyl steht und

m für die Zahlen 0, 1, 2 oder 3 steht,

sowie deren Säureadditions-Salze und Metallsalz-Komplexe.

**2.** Verfahren zur Herstellung von Hydroxyalkyltriazolyl-Derivaten der Formel

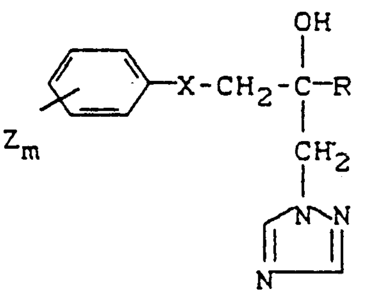

(I)

in welcher

R für die Reste der Formeln $-CH_2-CH(CH_3)_2$, $-(CH_2)_4-CH_3$,

$-(CH_2)_5-CH_3$, $-(CH_2)_6-CH_3$, 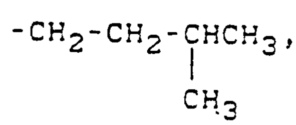,

$-(CH_2)_8CH_3$.

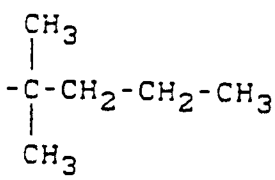 oder 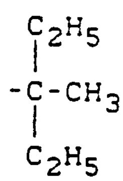 steht,

X für Sauerstoff oder Schwefel steht,

Z für Fluor, Chlor, Brom, Methyl, Ethyl, Trifluormethyl, Trifluormethoxy, Trifluormethylthio, Phenyl, Methoximinomethyl oder Ethoximinomethyl steht und

m für die Zahlen 0, 1, 2 oder 3 steht,

sowie von deren Säureadditions-Salzen und Metallsalz-Komplexen, dadurch gekennzeichnet, daß man Oxirane der Formel

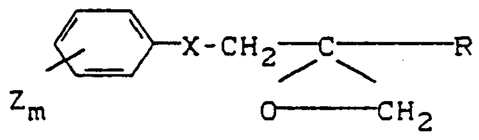

(II)

in welcher
R, X, Z und m die oben angegebene Bedeutung haben,
mit 1,2,4-Triazol der Formel

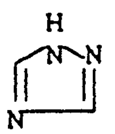

(III)

in Gegenwart eins Verdünnungsmittels und gegebenenfalls in Gegenwart eines Säurebindemittels sowie gegebenenfalls in Gegenwart eines Katalysators umsetzt und gegebenenfalls anschließend an die so erhaltenen Verbindungen der Formel (I) eine Säure oder ein Metallsalz addiert.

**3.** Fungizide und pflanzenwuchsregulierende Mittel, gekennzeichnet durch einen Gehalt an mindestens einem Hydroxyalkyl-triazolyl-Derivat der Formel (I) gemäß Anspruch 1 bzw. an einem Säureadditions-Salz oder Metallsalz-Komplex eines Hydroxyalkyl-triazolyl-Derivates der Formel (I).

**4.** Verfahren zur Bekämpfung von Pilzen und zur Regulierung des Pflanzenwachstums, dadurch gekennzeichnet, daß man Hydroxyalkyl-triazolyl-Derivate der Formel (I) gemäß Anspruch 1 bzw. deren Säureadditions-Salze oder Metallsalz-Komplexe auf die Pflanzen und/oder deren Lebensraum ausbringt.

**5.** Verwendung von Hydroxyalkyl-triazolyl-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

**6.** Verfahren zur Herstellung von fungiziden und pflanzenwuchsregulierenden Mitteln, dadurch gekennzeichnet, daß man Hydroxyalkyl-triazolyl-Derivate der Formel (I) gemäß Anspruch 1 bzw. deren Säureadditionssalze oder Metallsalz-Komplexe mit Streckmitteln und/oder oberflächenaktiven Stoffen vermischt,

**7.** Oxirane der Formel

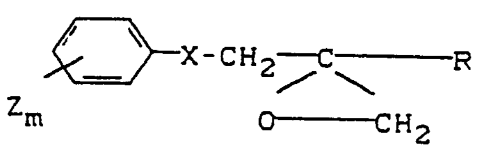

(II)

in welcher

R für die Reste der Formeln $-CH_2-CH(CH_3)_2$, $-(CH_2)_4-CH_3$,

$-(CH_2)_5-CH_3$, $-(CH_2)_6-CH_3$, 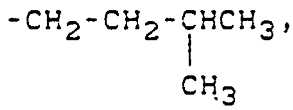,

$-(CH_2)_8CH_3$.

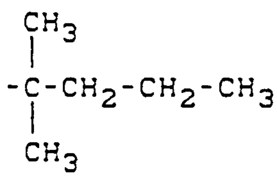 oder 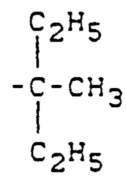 steht,

X für Sauerstoff oder Schwefel steht,
Z für Fluor, Chlor, Brom, Methyl, Ethyl, Trifluormethyl, Trifluormethoxy, Trifluormethylthio, Phenyl, Methoximinomethyl oder Ethoximinomethyl steht und
m für die Zahlen 0, 1, 2 oder 3 steht.

**8.** Verfahren zur Herstellung von Oxiranen der Formel

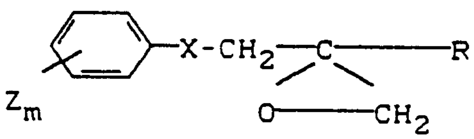 (II)

in welcher
R für die Reste der Formeln $-CH_2-CH(CH_3)_2$, $-(CH_2)_4-CH_3$,

$-(CH_2)_5-CH_3$, $-(CH_2)_6-CH_3$, 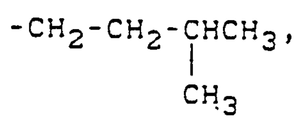,

$-(CH_2)_8CH_3$.

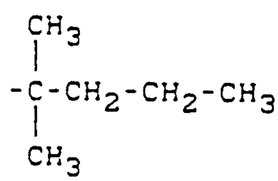 oder 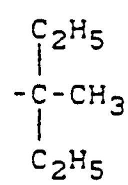 steht,

X für Sauerstoff oder Schwefel steht,
Z für Fluor, Chlor, Brom, Methyl, Ethyl, Trifluormethyl, Trifluormethoxy, Trifluormethylthio, Phenyl, Methoximinomethyl oder Ethoximinomethyl steht und
m für die Zahlen o, 1, 2 oder 3 steht,
dadurch gekennzeichnet, daß man

a) in einer ersten Stufe Ketone der Formel

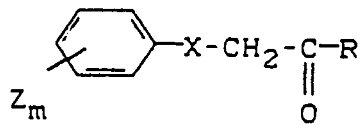

(IV)

in welcher
R, X, Z und m die oben angegebene Bedeutung haben,
mit Methyl-triphenyl-phosphoniumbromid der Formel

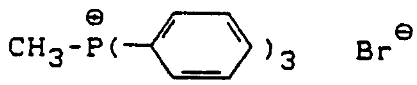

(V)

in Gegenwart einer Base und in Gegenwart eines Verdünnungsmittels umsetzt und dann in einer zweiten Stufe die so erhaltenen Verbindungen der Formel

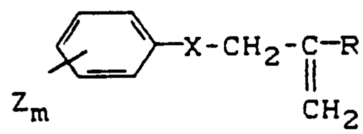

(VI)

in welcher
R, X, Z und m die oben angegebene Bedeutung haben,
mit Persäuren in Gegenwart eines Verdünnungsmittels umsetzt,

oder

b) Ketone der Formel

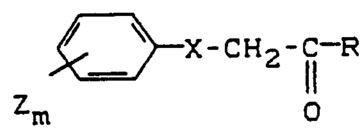

(IV)

in welcher
R, X, Z und m die oben angegebene Bedeutung haben,
entweder

α) mit Dimethyloxosulfonium-methylid der Formel

$$(CH_3)_2\overset{\delta^{\ominus}}{S}O\text{-}\overset{\delta^{\ominus}}{C}H_2 \qquad \text{(VII)}$$

oder

β) mit Dimethylsulfonium-methylid der Formel

$$(CH_3)_2\overset{\delta^{\ominus}}{S}\text{—}\overset{\delta^{\ominus}}{C}H_2 \qquad \text{(VIII)}$$

in Gegenwart eines Verdünnungsmittels umsetzt.

**9.** Verbindungen der Formel

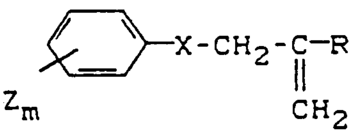

(VI)

in welcher

R für die Reste der Formeln $-CH_2-CH(CH_3)_2$, $-(CH_2)_4-CH_3$,

$-(CH_2)_5-CH_3$, $-(CH_2)_6-CH_3$, 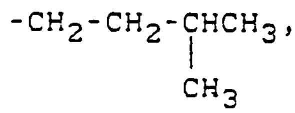

$-(CH_2)_8CH_3$

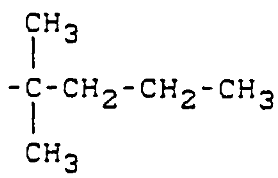 oder 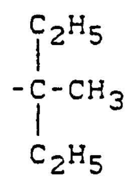 steht,

X für Sauerstoff oder Schwefel steht,

Z für Fluor, Chlor, Brom, Methyl, Ethyl, Trifluormethyl, Trifluormethoxy, Trifluormethylthio, Phenyl, Methoximinomethyl oder Ethoximinomethyl steht und

m für die Zahlen 0, 1, 2 oder 3 steht.

## Claims

**1.** Hydroxyalkyl-triazolyl derivatives of the formula

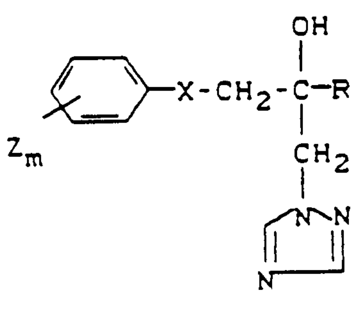

(I)

in which

R represents the radicals of the formulae $-CH_2-CH(CH_3)_2$, $-(CH_2)_4-CH_3$,

$-(CH_2)_5-CH_3$, $-(CH_2)_6-CH_3$, 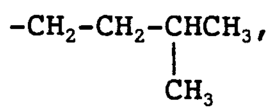

$-(CH_2)_8CH_3$,

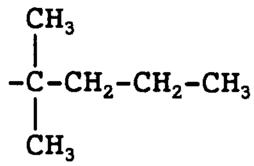 or 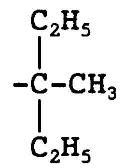 ,

X represents oxygen or sulphur,
Z represents fluorine, chlorine, bromine, methyl, ethyl, trifluoromethyl, trifluoromethoxy, trifluoromethylthio, phenyl, methoxyiminomethyl or ethoxyiminomethyl and
m represents the numbers 0, 1, 2 or 3,

and their acid addition salts and metal salt complexes.

**2.** Process for the preparation of hydroxyalkyl-triazolyl derivatives of the formula

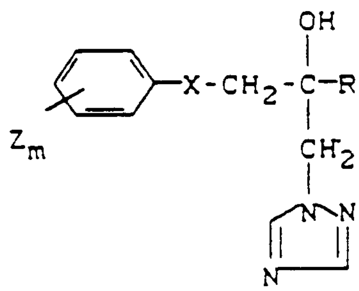

(I)

in which
R represents the radicals of the formulae $-CH_2-CH(CH_3)_2$, $-(CH_2)_4-CH_3$,

$-(CH_2)_5-CH_3$, $-(CH_2)_6-CH_3$, 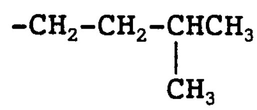 ,

$-(CH_2)_8CH_3$,

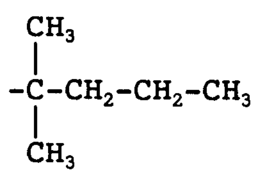 or 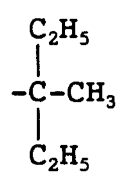 ,

X represents oxygen or sulphur,
Z represents fluorine, chlorine, bromine, methyl, ethyl, trifluoromethyl, trifluoromethoxy, trifluoromethylthio, phenyl, methoxyiminomethyl or ethoxyiminomethyl and
m represents the numbers 0, 1, 2 or 3,

and of their acid addition salts and metal salt complexes, characterised in that oxiranes of the formula

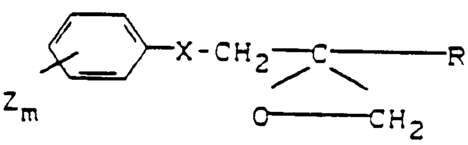

(II)

in which
R, X, Z and m have the abovementioned meaning,
are reacted with 1,2,4-triazole of the formula

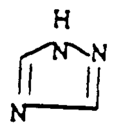

(III)

in the presence of a diluent and if appropriate in the presence of an acid binding agent and if appropriate in the presence of a catalyst, and if appropriate an acid or a metal salt is then added to the compounds of the formula (I) thus obtained.

**3.** Fungicides and plant growth regulating agents, characterised in that they contain at least one hydroxyalkyl-triazolyl derivative of the formula (I) according to Claim 1 or an acid addition salt or metal salt complex of a hydroxyalkyl-triazolyl derivative of the formula (I).

**4.** Method of combating fungi and regulating plant growth, characterised in that hydroxyalkyl-triazolyl derivatives of the formula (I) according to Claim 1 or their acid addition salts or metal salt complexes are applied to the plants and/or their environment.

**5.** Use of hydroxyalkyl-triazolyl derivatives of the formula (I) according to Claim 1 or their acid addition salts and metal salt complexes for combating fungi and for regulating plant growth.

**6.** Process for the preparation of fungicidal and plant growth regulating agents, characterised in that hydroxyalkyl-triazolyl derivatives of the formula (I) according to Claim 1 or their acid addition salts or metal salt complexes are mixed with extenders and/or surface-active substances.

**7.** Oxiranes of the formula

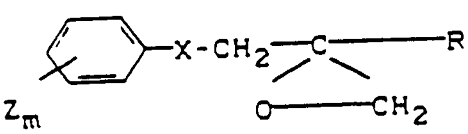

(II)

in which
R represents the radicals of the formulae $-CH_2-CH(CH_3)_2$, $-(CH_2)_4-CH_3$,

$$-(CH_2)_5-CH_3,\ -(CH_2)_6-CH_3,\ -CH_2-CH_2-\underset{\displaystyle CH_3}{\underset{|}{C}}HCH_3,$$

$-(CH_2)_8CH_3$,

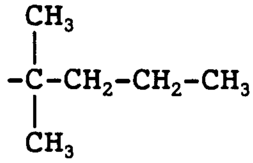 or 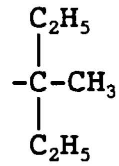 ,

X represents oxygen or sulphur,

Z represents fluorine, chlorine, bromine, methyl, ethyl, trifluoromethyl, trifluoromethoxy, trifluoromethylthio, phenyl, methoxyiminomethyl or ethoxyiminomethyl and

m represents the numbers 0, 1, 2 or 3.

**8.** Process for the preparation of oxiranes of the formula

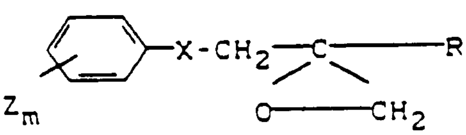

(II)

in which

R represents the radicals of the formulae $-CH_2-CH(CH_3)_2$, $-(CH_2)_4-CH_3$,

$-(CH_2)_5-CH_3$, $-(CH_2)_6-CH_3$, 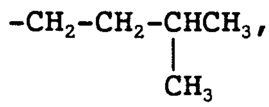

$-(CH_2)_8CH_3$ ,

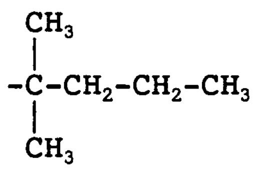 or 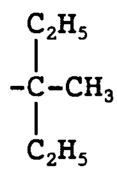 ,

X represents oxygen or sulphur,

Z represents fluorine, chlorine, bromine, methyl, ethyl, trifluoromethyl, trifluoromethoxy, trifluoromethylthio, phenyl, methoxyiminomethyl or ethoxyiminomethyl and

m represents the numbers 0, 1, 2 or 3,

characterised in that

a) in a first step, ketones of the formula

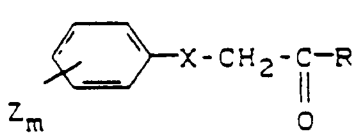

(IV)

in which

R, X, Z and m have the abovementioned meaning,

are reacted with methyl-triphenyl-phosphonium bromide of the formula

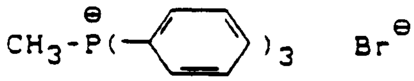

(V)

in the presence of a base and in the presence of a diluent, and then, in a second step, the compounds thus obtained of the formula

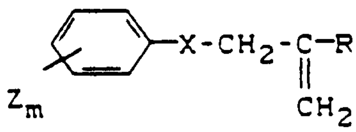

(VI)

in which
R, X, Z and m have the abovementioned meaning,
are reacted with peracids in the presence of a diluent,
or
b) ketones of the formula

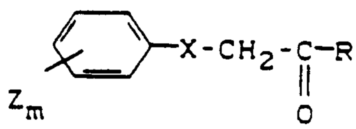

(IV)

in which
R, X, Z and m have the abovementioned meaning,
are reacted either
α) with dimethyloxosulphonium methylide of the formula

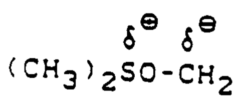

(VII)

or
β) with dimethylsulphonium methylide of the formula

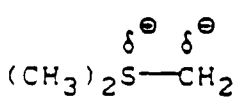

(VIII)

in the presence of a diluent.

**9.** Compounds of the formula

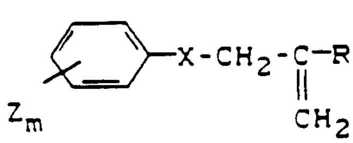

(VI)

in which

R represents the radicals of the formulae $-CH_2-CH(CH_3)_2$, $-(CH_2)_4-CH_3$ ,

$-(CH_2)_5-CH_3$, $-(CH_2)_6-CH_3$, 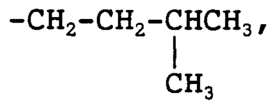

$-(CH_2)_8CH_3$ ,

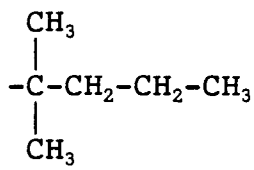 or 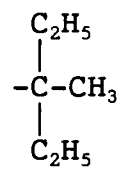 ,

X represents oxygen or sulphur,

Z represents fluorine, chlorine, bromine, methyl, ethyl, trifluoromethyl, trifluoromethoxy, trifluoromethylthio, phenyl, methoxyiminomethyl or ethoxyiminomethyl and

m represents the numbers 0, 1, 2 or 3.

## Revendications

**1.** Dérivés d'hydroxyalkyl-triazoles de formule

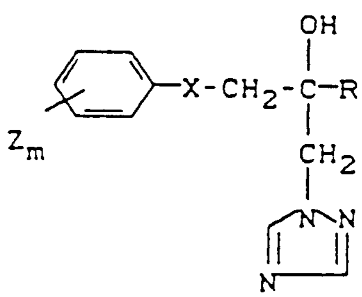

(I)

dans laquelle

R représente les restes de formules $-CH_2-CH(CH_3)_2$, $-(CH_2)_4-CH_3$,

$-(CH_2)_5-CH_3$, $-(CH_2)_6-CH_3$, 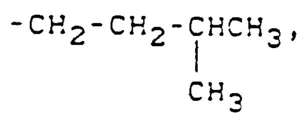

$-(CH_2)_8CH_3$

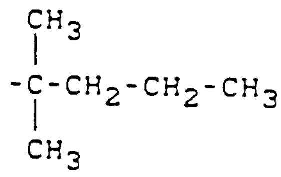

ou

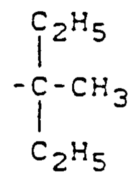

X représente l'oxygène ou le soufre,
Z représente le fluor, le chlore, le brome, un groupe méthyle, éthyle, trifluorométhyle, trifluorométhoxy, trifluorométhylthio, phényle, méthoximinométhyle ou éthoximinométhyle et
m représente les nombres 0, 1, 2 ou 3,

ainsi que leurs sels d'addition d'acides et leurs complexes de sels métalliques.

**2.** Procédé de production de dérives d'hydroxyalkyl-triazoles de formule

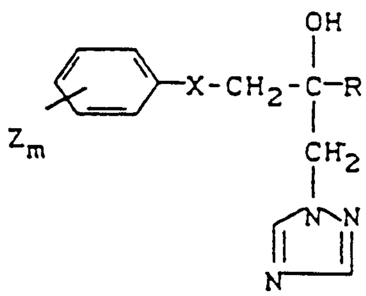

(I)

dans laquelle

R représente les restes de formules $-CH_2-CH(CH_3)_2$, $-(CH_2)_4-CH_3$,

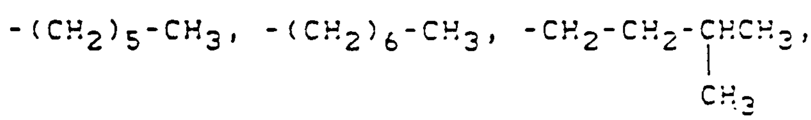

$-(CH_2)_8CH_3$

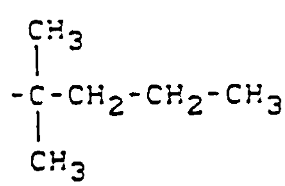

ou

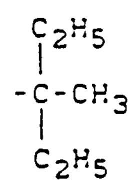

X est l'oxygène ou le soufre,
Z représente le fluor, le chlore, le brome, un groupe méthyle, éthyle, trifluorométhyle, trifluorométhoxy, trifluorométhylthio, phényle, méthoximinométhyle ou éthoximinométhyle et
m représente les nombres 0, 1, 2 ou 3,

ainsi que de leurs sels d'addition d'acides et de leurs complexes de sels métalliques, caractérisé en ce qu'on fait réagir des oxirannes de formule

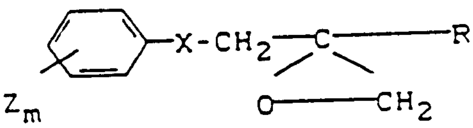

(II)

dans laquelle
R, X, Z et m ont la définition indiquée ci-dessus, avec le 1,2,4-triazole de formule

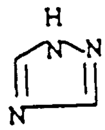

(III)

en présence d'un diluant et, le cas échéant, en présence d'un accepteur d'acide, ainsi qu'en la présence éventuelle d'un catalyseur, et on additionne ensuite éventuellement sur les composés de formule (I) ainsi obtenus un acide ou un sel métallique.

**3.** Compositions fongicides et régulatrices de croissance des plantes, caractérisées par une teneur en au moins un dérivé d'hydroxyalkyl-triazole de formule (I) suivant la revendication 1 ou d'un sel d'addition d'acide ou d'un complexe de sel métallique d'un dérivé d'hydroxyalkyl-triazole de formule (I).

**4.** Procédé pour combattre des champignons et pour influencer la croissance des plantes, caractérisé en ce qu'on épand des dérivés d'hydroxyalkyl-triazoles de formule (I) suivant la revendication 1 ou leurs sels d'addition d'acides ou leurs complexes de sels métalliques sur les plantes et/ou sur leur milieu.

**5.** Utilisation de dérivés d'hydroxyalkyltriazoles de formule (I) suivant la revendication 1 ou de leurs sels d'addition d'acides et de leurs complexes de sels métalliques pour combattre des champignons ainsi que pour la régulation de la croissance des plantes.

**6.** Procédé de préparation de compositions fongicides et régulatrices de croissance des plantes, caractérisé en ce qu'on mélange des dérivés d'hydroxyalkyltriazoles de formule (I) suivant la revendication 1 ou leurs sels d'addition d'acides ou leurs complexes de sels métalliques avec des diluants et/ou des agents tensioactifs.

**7.** Oxirannes de formule

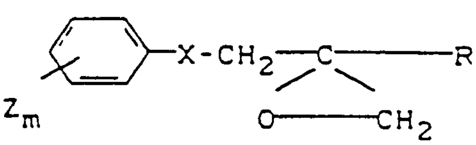

(II)

dans laquelle
R représente les restes de formules $-CH_2-CH(CH_3)_2$, $-(CH_2)_4-CH_3$,

$-(CH_2)_5-CH_3$, $-(CH_2)_6-CH_3$, 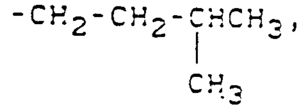

$-(CH_2)_8CH_3$

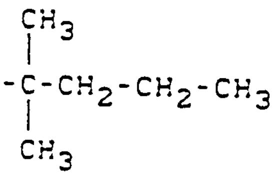 ou 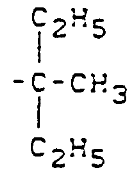

X représente l'oxygène ou le soufre,

Z est le fluor, le chlore, le brome, un groupe méthyle, éthyle, trifluorométhyle, trifluorométhoxy, trifluorométhylthio, phényle, méthoximinométhyle ou éthoxyiminométhyle et

m représente les nombres 0, 1, 2 ou 3.

**8.** Procédé de production d'oxirannes de formule

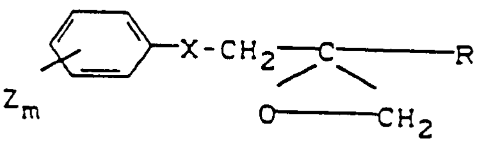

(II)

dans laquelle

R représente les restes de formules $-CH_2-CH(CH_3)_2$, $-(CH_2)_4-CH_3$,

$-(CH_2)_5-CH_3$, $-(CH_2)_6-CH_3$, 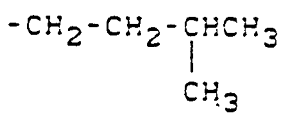,

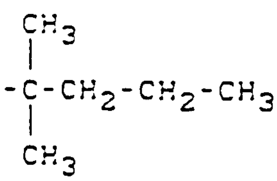 ou 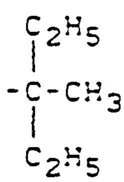

X représente l'oxygène ou le soufre,

Z représente le fluor, le chlore, le brome, un groupe méthyle, éthyle, trifluorométhyle, trifluorométhoxy, trifluorométhylthio, phényle, méthoximinométhyle ou éthoximinométhyle et

m représente les nombres 0, 1, 2 ou 3,

caractérisé en ce que

a) on fait réagir dans une première étape des cétones de formule

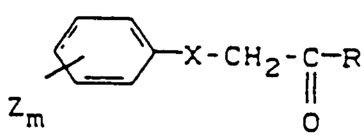

(IV)

dans laquelle

R, X, Z et m ont la définition indiquée ci-dessus, avec le bromure de méthyl-triphényl-phosphonium de formule

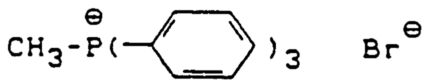

(V)

en présence d'une base et en présence d'un diluant, puis

dans une <u>seconde étape</u>, on fait réagir les composés ainsi obtenus de formule

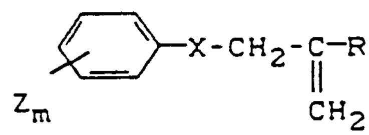

(VI)

dans laquelle

R, X, Z et m ont la définition indiquée ci-dessus avec des peracides en présence d'un diluant, ou bien

b) on fait réagir des cétones de formule

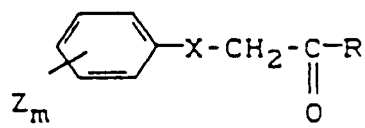

(IV)

dans laquelle

R, X, Z et m ont la définition indiquée ci-dessus, ou bien

α) avec le méthylure de diméthyloxosulfonium de formule

$$(CH_3)_2\overset{\delta^{\ominus}}{S}O-\overset{\delta^{\ominus}}{C}H_2 \qquad (VII)$$

ou bien

β) avec le méthylure de diméthylsulfonium de formule

$$(CH_3)_2\overset{\delta^{\ominus}}{S}-\overset{\delta^{\ominus}}{C}H_2 \qquad (VIII)$$

en présence d'un diluant.

**9.** Composés de formule

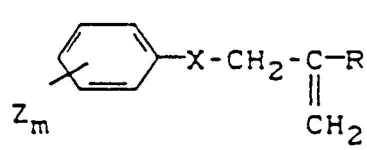

(VI)

dans laquelle

R représente les restes de formules $-CH_2-CH(CH_3)_2$, $-(CH_2)_4-CH_3$,

$-(CH_2)_5-CH_3$, $-(CH_2)_6-CH_3$, 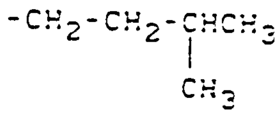 ,

$-(CH_2)_8CH_3$

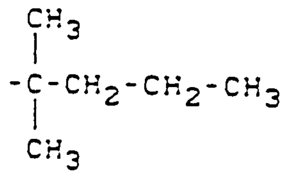 ou 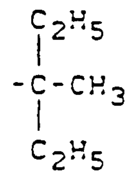

X représente l'oxygène ou le soufre,

Z est le fluor, le chlore, le brome, un groupe méthyle, éthyle, trifluorométhyle, trifluorométhoxy, trifluorométhylthio, phényle, méthoximinométhyle ou éthoximinométhyle et

m représente les nombres 0, 1, 2 ou 3.